# EUROPÄISCHE PATENTANMELDUNG

(11) **EP 0 820 759 A2**
(43) Veröffentlichungstag der Anmeldung: **28.01.1998**
(21) Anmeldenummer: 97112194.2
(22) Anmeldetag: 17.07.1997
(51) Int. Cl.: A61K 7/13

(54) **Mittel zum Färben von keratinhaltigen Fasern**

(30) Priorität: 26.07.1996 DE 19630275
(71) Anmelder: Henkel Kommanditgesellschaft auf Aktien, 40589 Düsseldorf-Holthausen (DE)
(72) Erfinder: Möller, Hinrich, Dipl.-Chem. Dr., 40789 Monheim (DE); Höffkes, Horst, Dipl.-Chem. Dr., 40595 Düsseldorf (DE)

(57) **Zusammenfassung**

Mittel zum Färben von keratinhaltigen Fasern, insbesondere menschlichen Haaren, enthaltend
a) einen Aldehyd der Formel I 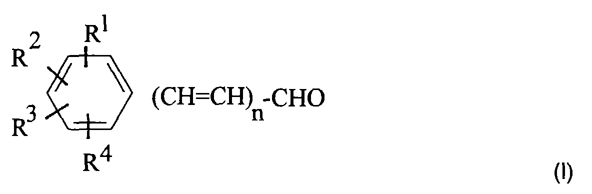 in der R¹, R², R³ und R⁴ unabhängig voneinander für Wasserstoffatome, Halogenatome, C₁-C₄-Alkyl-, C₂-C₄-Hydroxyalkyl-, Amino-C₁-C₄-alkyl-, C₁-C₄-Alkoxy-, Nitro-, Carboxy-, C₁-C₄-Acylamino-, Sulfo-, Hydroxy- oder Aminogruppen, die durch C₁-C₄-Alkyl- oder C₂-C₄-Hydroxyalkylgruppen substituiert sein können, stehen, und n für 0 oder 1 steht, und
b) ein Oxidationsfarbstoffvorprodukt, das ein primäres oder sekundäres aromatisches Amin; eine stickstoffhaltige heterocyclische Verbindung; eine aromatische Hydroxyverbindung; eine Aminosäure, ein aus 2 bis 9 Aminosäuren aufgebautes Oligopeptid, oder ein Gemisch der voranstehenden ist,
   mit der Maßgabe, daß, wenn n gleich 0 ist, das Oxidationsfarbstoffvorprodukt ausgewählt ist aus der Gruppe 2-Aminomethyl-4-aminophenol, 1,8-Bis-(2,5-diaminophenoxy)-3,6-dioxaoctan, 2,4,5,6-Tetraaminopyrimidin, 2-Methylamino-3-amino-6-methoxypyridin, 2,6-Dimethoxy-3,5-diaminopyridin, 2-(2,5-Diaminophenyl)-ethanol und 1,3-Bis-(2,4-diaminophenoxy)-propan, Aminosäuren, aus 2 bis 9 Aminosäuren aufgebauten Oligopeptide, oder Gemischen der voranstehenden.

## Beschreibung

Gegenstand der Erfindung sind Mittel zum Färben von keratinhaltigen Fasern, insbesondere menschlichen Haaren, die Benz- oder Zimtaldehyde und Oxidationsfarbstoffvorprodukte enthalten.

Für das Färben von keratinhaltigen Fasern, z. B. Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Mit Oxidationsfarbstoffen lassen sich zwar intensive Färbungen mit guten Echtheitseigenschaften erzielen, die Entwicklung der Farbe geschieht jedoch i.a. unter dem Einfluß von Oxidationsmitteln wie z.B. H₂0₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

Gute Oxidationsfarbstoffvorprodukte müssen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufziehvermögen auf die Faser besitzen, wobei insbesondere bei menschlichen Haaren keine merklichen Unterschiede zwischen strapaziertem und frisch nachgewachsenem Haar bestehen dürfen (Egalisiervermögen). Sie sollen beständig sein gegen Licht, Wärme und den Einfluß chemischer Reduktionsmittel, z. B. gegen Dauerwellflüssigkeiten. Schließlich sollen sie - falls als Haarfärbemittel zur Anwendung kommend - die Kopfhaut nicht zu sehr anfärben, und vor allem sollen sie in toxikologischer und dermatologischer Hinsicht unbedenklich sein.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxy-4,5,6-triaminopyrimidin, 2,4-Dihydroxy-5,6-diaminopyrimidin und 2,5,6-Triaminohydroxypyrimidin.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 2-Chlor-resorcin, 4-Chlor-resorcin, 2-Chlor-6-methyl-3-aminophenol, 2-Methylresorcin und 5-Methylresorcin.

Bezüglich weiterer üblicher Farbstoffkomponenten wird ausdrücklich auf die Colipa-Liste, herausgegeben vom Industrieverband Körperpflege und Waschmittel, Frankfurt, Bezug genommen.

Die deutsche Auslegeschrift DE 28 30 497 offenbart Haarfärbemittel, die Salicylaldehyd in Kombination mit Oxidationsfarbstoffvorprodukten enthalten, z.B. 2,5-Diaminotoluol, 4-Aminophenol oder 2,4-Diaminotoluol, die jedoch (in Kombination mit dem zusätzlich enthaltenen Wasserstoffperoxid) ein mehr oder weniger großes Sensibilisierungspotential aufweisen können. Die deutsche Auslegeschrift DE 29 32 489 offenbart ebenfalls H₂0₂-haltige Haarfärbemittel, die Benzaldehyde, z.B. 2-Hydroxy-3-methoxybenzaldehyd oder 4-Hydroxy-3-methoxybenzaldehyd, in Kombination mit Oxidationsfarbstoffvorprodukten enthalten.

Die Patentschriften US 5,034,014 und US 5,199,954 offenbaren Haarfärberezeptur-Beispiele, die p-Dimethylaminobenzaldehyd oder p-Dimethylaminozimtaldehyd z.B. in Kombination mit dem sensibilisierend wirkenden p-Phenylendiamin enthalten.

Die Patentschrift US 4,391,603 hat oxidationsmittelfreie Haarfärbemittel zum Gegenstand, die substituierte Benzaldehyde enthalten. Die dort offenbarten Haarfärbemittel stellen direktziehende Haarfärbemittel dar, mit denen sich nicht die Farbnuancen, Farbtiefen und Farbechtheiten von Oxidationshaarfarbstoffvorprodukt-haltigen Haarfärbemitteln erreichen lassen.

Aufgabe der vorliegenden Erfindung ist es, Färbemittel für Keratinfasern, insbesondere menschliche Haare, bereitzustellen, die hinsichtlich der Farbtiefe, der Grauabdeckung und den Echtheitseigenschaften qualitativ mit üblichen Oxidationshaarfärbemitteln gleichwertig sind, ohne jedoch unbedingt auf Oxidationsmittel wie z.B. H₂0₂ angewiesen zu sein. Darüber hinaus sollten die Färbemittel kein oder nur ein sehr geringes Sensibilisierungspotential aufweisen.

Die Aufgabe wurde gelöst durch Bereitstellung eines Mittels zum Färben von keratinhaltigen Fasern, insbesondere menschlichen Haaren, enthaltend
a) mindestens einen Aldehyd der Formel I 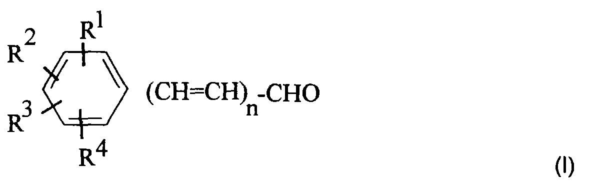 in der R¹, R², R³ und R⁴ unabhängig voneinander für Wasserstoffatome, Halogenatome, C₁-C₄-Alkyl-, C₂-C₄-Hydroxyalkyl-, Amino-C₁-C₄-alkyl-, C₁-C₄-Alkoxy-, Nitro-, Carboxy-, C₁-C₄-Acylamino-, Sulfo-, Hydroxy- oder Aminogruppen, die durch C₁-C₄-Alkyl- oder C₂-C₄-Hydroxyalkylgruppen substituiert sein können, stehen, und n für 0 oder 1 steht, und
b) mindestens ein Oxidationsfarbstoffvorprodukt, das ein primäres oder sekundäres aromatisches Amin, eine stickstoffhaltige heterocyclische Verbindung, eine aromatische Hydroxyverbindung; eine Aminosäure, ein aus 2 bis 9 Aminosäuren aufgebautes Oligopeptid, oder ein Gemisch der voranstehenden ist,
mit der Maßgabe, daß, wenn n gleich 0 ist, das Oxidationsfarbstoffvorprodukt ausgewählt ist aus der Gruppe 2-Aminomethyl-4-aminophenol, 1,8-Bis-(2,5-diaminophenoxy)-3,6-dioxaoctan, 2,4,5,6-Tetraaminopyrimidin, 2-Methylamino-3-amino-6-methoxypyridin, 2,6-Dimethoxy-3,5-diaminopyridin, 2-(2,5-Diaminophenyl)-ethanol und 1,3-Bis-(2,4-diaminophenoxy)-propan, Aminosäuren, aus 2 bis 9 Aminosäuren aufgebauten Oligopeptide, oder Gemischen der voranstehenden. Bevorzugte Aminosäuren sind Arginin, Histidin, Tyrosin, Phenylalanin, DOPA (Dihydroxyphenyldialanin), Ornithin, Lysin und Tryptophan.

Als keratinhaltige Fasern kommen z.B. Wolle, Pelze, Federn und vor allem menschliche Haare in Betracht. Die erfindungsgemäßen Haarfärbemittel können prinzipiell aber auch zum Färben anderer Naturfasern, wie z.B. Baumwolle, Jute, Sisal, Leinen oder Seide, modifizierter Naturfasern, wie z.B. Regeneratcellulose, Nitro-, Alkyl- oder Hydroxyalkyl- oder Acetylcellulose und synthetischer Fasern, wie z.B. Polyamid-, Polyacrylnitril-, Polyurethan- und Polyesterfasern verwendet werden.

Die Aldehyde der Formel I sowie die Oxidationsfarbstoffvorprodukte sind vorzugsweise in einer Menge von jeweils 0,03 bis 65, insbesondere 1 bis 40 mmol, jeweils bezogen auf 100 g des gesamten Färbemittels, enthalten.

Der Aldehyd der Formel I ist dabei vorzugsweise ausgewählt aus der Gruppe 4-Dimethylaminozimtaldehyd, 4-Dimethylaminobenzaldehyd, 2-Hydroxybenzaldehyd (Salicylaldehyd), 4-Hydroxy-3-methoxybenzaldehyd (Vanillin), 2,3,4-Trihydroxybenzaldehyd, 4-Diethylamino-2-hydroxybenzaldehyd und 3-Methoxy-4-hydroxyzimtaldehyd (Coniferylaldehyd).

Das als Oxidationsfarbstoffvorprodukt eingesetzte primäre oder sekundäre aromatische Amin ist vorzugsweise ausgewählt aus der Gruppe bestehend aus N-(2-Hydroxyethyl)-N-ethyl-, N-(2-Methoxyethyl-), 2,3-, 2,4-, 2,5-Dichlor-p-phenylendiamin, 2-Chlor-p-phenylendiamin, 2,5-Dihydroxy-4-morpholinoanilin-dihydrobromid, 2-, 3-Aminophenol, 2-Aminomethyl-4-aminophenol, o-, m-Phenylendiamin, 2,5-Diaminotoluol, -phenol, -anisol, -phenethol, 2-(2,5-Diaminophenyl)-ethanol, 2-Chlor-p-phenylendiamin, 4-Methylamino-, 3-, 4-Dimethylamino-, 3,4-Methylendioxyanilin, 3-Amino-2,4-dichlor-, 4-Methylamino-, 2-Methyl-5-amino-, 3-Methyl-4-amino-, 2-Methyl-5-(2-hydroxyethylamino)-, 2-Methyl-5-amino-6-chlor-, 2-Methyl-5-amino-4-chlor-, 2-Methyl-5-amino-6-chlor-, 5-(2-Hydroxyethylamino)-4-methoxy-2-methyl-, 4-Amino-2-aminomethyl-phenol, 1,3-Diamino-2,4-dimethoxybenzol, 2-, 3-, 4-Aminobenzoesäure, -phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxy-benzoesäure, 2-, 3-, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesaure, 3-Aminophthalsäure, 5-Aminoisophthalsäure, 1,3,5-, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 3,5-Diamino-4-hydroxybrenzcatechin, aromatische Aniline bzw. Phenole mit einem weiteren aromatischen Rest wie 4,4'-Diaminostilben, 4,4'-Diaminostilben-2,2'-disulfonsäure, Na-Salz, 4,4'-Diaminodiphenylmethan, -sulfid, -sulfoxid, -amin, 4,4'-Diaminodiphenylamin-2-sulfonsäure, 4,4'-Diaminobenzophenon, -diphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)-propan, -2-propanol, 1,3-Bis-[N-(4-aminophenyl)-2-hydroxyethylamino]-2-propanol, N,N-Bis-[2-(4-aminophenoxy)-ethyl]methylamin und sowie die vorzugsweise mit anorganischen Säuren gebildeten physiologisch verträglichen Salze, wie z.B. Chloride und Sulfate.

Als stickstoffhaltige heterocyclische Verbindungen kommen beispielsweise solche in Betracht, die ausgewählt sind aus der Gruppe aus 2-, 3-, 4-Amino-, 2-Amino-3-hydroxy-, 2,6-Diamino-, 2,5-Diamino-, 2,3-Diamino-, 2-Dimethylamino-5-amino-, 2-Methylamino-3-amino-6-methoxy-, 2,3-Diamino-6-methoxy-, 2,6-Dimethoxy-3,5-diamino-, 2,4,5-Triamino-, 2,6-Dihydroxy-3,4-dimethylpyridin, 4,5,6-Triamino-, 4-Hydroxy-2,5,6-triamino-, 2,4,5,6-Tetraamino-, 2-Methylamino-4,5,6-triamino-, 2,4-, 4,5-Diamino-, 2-Amino-4-methoxy-6-methyl-pyrimidin, 3,5-Diaminopyrazol, -1,2,4-triazol, 3-Amino-, 3-Amino-5-hydroxypyrazol, 2-,3-, 8-Aminochinolin, 4-Amino-chinaldin, 2-, 6-Aminonicotinsäure, 5-Aminoisochinolin, 4-, 5-, 6-, 7-Aminoindol, 5-, 6-Aminoindazol, 5-, 7-Amino-benzimidazol, -benzothiazol, 2,5-Dihydroxy-4-morpholinoanilin sowie Indol- und Indolinderivaten, wie 5,6-Dihydroxyindol, 5,6-Dihydroxyindolin und 4-Hydroxyindolin. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Geeignete aromatische Hydroxyverbindungen sind z.B. 2-, 4-, 5-Methylresorcin, Resorcin, 3-Methoxyphenol, Brenzkatechin, Hydrochinon, Pyrogallol, Phloroglucin, Hydroxyhydrochinon,
2-, 3-, 4-Methoxy-, 3-Dimethylamino-, 2-(2-Hydroxyethyl)-, 3,4-Methylendioxyphenol, 2,4-, 3,4-Dihydroxybenzoesäure, -phenylessigsäure, Gallussäure, 2,4,6-Trihydroxybenzoesäure, -acetophenon, 2-, 4-Methyl-, 2-, 4-Chlorresorcin, 1-, 2-Naphthol, 1,5-, 2,3-, 2,7-Dihydroxynaphthalin, 6-Dimethylamino-4-hydroxy-2-naphthalinsulfonsäure, 3,6-Dihydroxy-2,7-naphthalinsulfonsäure.

Als Aminosäuren kommen alle natürlich vorkommenden und synthetischen Aminosäuren in Frage, z.B. die durch Hydrolyse aus pflanzlichen oder tierischen Proteinen, z.B. Kollagen, Keratin, Casein, Elastin, Sojaprotein, Weizengluten oder Mandelprotein zugänglichen Aminosäuren. Dabei können sowohl sauer als auch alkalisch reagierende Aminosäuren eingesetzt werden.

Die Oligopeptide können dabei natürlich vorkommende oder synthetische Oligopeptide, aber auch die in Polypeptid- oder Proteinhydrolysaten enthaltenen Oligopeptide sein, sofern sie über eine für die Anwendung in den erfindungsgemäßen Färbemitteln ausreichende Wasserlöslichkeit verfügen. Als Beispiele sind z.B. Glutathion oder die in den Hydrolysaten von Kollagen, Keratin, Gasen, Elastin, Sojaprotein, Weizengluten oder Mandelprotein enthaltenen Oligopeptide zu nennen.

Die Gruppe der voranstehend genannten Oxidationsfarbstoffvorprodukte umfaßt sowohl bekannte Kupplerkomponenten als auch Entwicklerkomponenten.

Ein weiterer Gegenstand der vorliegenden Erfindung ist die Verwendung der vorgenannten Aldehyde der Formel I als eine färbende Komponente in Oxidationshaarfärbemitteln.

In einer bevorzugten Ausführungsform enthalten die erfindungsgemäßen Haarfärbemittel zur weiteren Modifizierung der Farbnuancen neben den Oxidationsfarbstoffvorprodukten zusätzlich übliche direktziehende Farbstoffe, z. B. aus der Gruppe der Nitrophenylendiamine, Nitroaminophenole, Anthrachinone oder Indophenole, wie z.B. die unter den internationalen Bezeichnungen bzw. Handelsnamen HC Yellow 2, HC Yellow 4, Basic Yellow 57, Disperse Orange 3, HC Red 3, HC Red BN, Basic Red 76, HC Blue 2, Disperse Blue 3, Basic Blue 99, HC Violet 1, Disperse Violet 1, Disperse Violet 4, Disperse Black 9, Basic Brown 16, Pikraminsäure und Rodol 9 R bekannten Verbindungen sowie 4-N-Ethyl-1,4-bis-(2 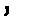 -hydroxyethyl-amino)-2-nitrobenzol, 1-(2 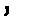 -Hydroxyethyl)-amino-4-methyl-2-nitro-benzol, 1-Amino-2-nitro-4-(2 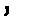 -hydroxyethylamino)-5-chlorbenzol, 4-Amino-2-nitro-diphenylamin-2 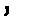 -carbonsäure und 6-Nitro-1,2,3,4-tetrahydrochinoxalin in einer Menge von 0,01 bis 20 Gew.-%, bezogen auf das gesamte Oxidationshaarfärbemittel.

Es ist nicht erforderlich, daß die Oxidationsfarbstoffvorprodukte oder die fakultativ enthaltenen direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Auf die Anwesenheit von Oxidationsmitteln, z.B. H₂0₂, kann in der vorliegenden Erfindung verzichtet werden. Es kann jedoch u.U. wünschenswert sein, den erfindungsgemäßen Mitteln zur Erzielung von Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist H₂O₂.

Die erfindungsgemäßen Färbemittel ergeben eine breite Palette von Farbnuancen im Bereich von gelborange bis braunschwarz; die Echtheitseigenschaften sind hervorragend, die Sensibilisierungspotentiale sehr gering.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel in einen wasserhaltigen kosmetischen Träger eingearbeitet werden. Geeignete wasserhaltige kosmetische Träger sind z.B. Cremes, Emulsionen, Gele oder auch tensidhaltige schäumende Lösungen wie z.B. Shampoos oder andere Zubereitungen, die für die Anwendung auf den keratinhaltigen Fasern geeignet sind.

In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammoniumglycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilsiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Dialkylammoniummethosulfate und Methyl-hydroxyalkyldialkoyloxyalkyl-ammoniummethosulfate.

Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert.-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Farbstoffe zum Einfärben der Zubereitungen,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammonium- oder Metallsalze zuzugeben. Geeignete Metallsalze sind z.B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Erdalkalimetallen, wie Kalium, Natrium oder Lithium, Erdalkalimetallen, wie Magnesium, Calcium, Strontium oder Barium, oder von Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Amoniumcarbonat, -chlorid und -acetat bevorzugt sind. Diese Salze sind vorzugsweise in einer Menge von 0,03 bis 65, insbesondere von 1 bis 40 mmol, bezogen auf 100 g des gesamten Färbemittels, enthalten.

Der pH-Wert der gebrauchsfertigen Färbezubereitungen liegt üblicherweise zwischen 2 und 11, vorzugsweise zwischen 5 und 9.

Ein weiterer Gegenstand der vorliegenden Erfindung ist ein Verfahren zum Färben von keratinhaltigen Fasern, worin keratinhaltige Fasern mit einem Oxidationsfärbemittel, enthaltend
a) mindestens einen Aldehyd der Formel I 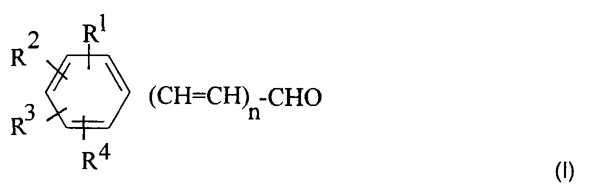 in der R¹, R², R³ und R⁴ unabhängig voneinander für Wasserstoffatome, Halogenatome, C₁-C₄-Alkyl-, C₂-C₄-Hydroxyalkyl-, Amino-C₁-C₄-alkyl-, C₁-C₄-Alkoxy-, Nitro-, Carboxy-, C₁-C₄-Acylamino-, Sulfo-, Hydroxy- oder Aminogruppen, die durch C₁-C₄-Alkyl- oder C₂-C₄-Hydroxyalkylgruppen substituiert sein können, stehen, und n für 0 oder 1 steht, und
b) mindestens ein Oxidationsfarbstoffvorprodukt, das ein primäres oder sekundäres aromatisches Amin; eine stickstoffhaltige heterocyclische Verbindung; eine aromatische Hydroxyverbindung; eine Aminosäure, ein aus 2 bis 9 Aminosäuren aufgebautes Oligopeptid, oder ein Gemisch der voranstehenden ist,
mit der Maßgabe, daß, wenn n gleich 0 ist, das Oxidationsfarbstoffvorprodukt ausgewählt ist aus der Gruppe 2-Aminomethyl-4-aminophenol, 1,8-Bis-(2,5-diaminophenoxy)-3,6-dioxaoctan, 2,4,5,6-Tetraaminopyrimidin, 2-Methylamino-3-amino-6-methoxypyridin, 2,6-Dimethoxy-3,5-diaminopyridin, 2-(2,5-Diaminophenyl)-ethanol und 1,3-Bis-(2,4-diaminophenoxy)-propan, Aminosäuren, aus 2 bis 9 Aminosäuren aufgebauten Oligopeptide, oder Gemischen der voranstehenden, ggf. im Gemisch mit einem wasserlöslichen Träger und weiteren Wirk-, Zusatz- und Hilfsstoffen auf die keratinhaltigen Fasern aufgebracht aufgebracht, einige Zeit, üblicherweise ca. 30 Minuten, auf der Faser belassen und anschließend wieder ausgespült oder mit einem handelsüblichen Haarshampoo ausgewaschen wird.

Die Aldehyde der Formel I und die Oxidationsfarbstoffvorprodukte können entweder gleichzeitig auf das Haar aufgebracht werden oder aber auch nacheinander, wobei es unerheblich ist, welche der beiden Komponenten zuerst aufgetragen wird. Die fakultativ enthaltenen Ammonium- oder Metallsalze können dabei der ersten oder der zweiten Komponente zugesetzt werden. Zwischen dem Auftragen der ersten und der zweiten Komponente können bis zu 30 Minuten Zeitabstand liegen. Auch eine Vorbehandlung der Fasern mit der Salzlösung ist möglich.

Die Aldehyde der Formel I und die Oxidationsfarbstoffvorprodukte können entweder getrennt oder zusammen gelagert werden, entweder in einer flüssigen bis pastösen Zubereitung (wäßrig oder wasserfrei) oder als trockenes Pulver. Werden die Komponenten in einer flüssigen Zubereitung zusammen gelagert, so sollte diese zur Verminderung einer Reaktion der Komponenten weitgehend wasserfrei sein. Bei der getrennten Lagerung werden die reaktiven Komponenten erst unmittelbar vor der Anwendung miteinander innig vermischt. Bei der trockenen Lagerung wird vor der Anwendung üblicherweise eine definierte Menge warmen (50 bis 80°C) Wassers hinzugefügt und eine homogene Mischung hergestellt.

### Beispiele

### Herstellung einer Färbelösung

Es wurde eine Aufschlämmung von 10 mMol eines Aldehyds der Formel I, 10 mMol eines Oxidationsfarbstoffvorproduktes, 10 mMol Natriumacetat und ein Tropfen einer 20 %igen Fettalkylethersulfat-Lösung in 100 ml Wasser bereitet. Die Aufschlämmung wurde kurz auf ca. 80°Cerhitzt und nach dem Abkühlen filtriert, der pH-Wert wurde anschließend auf 6 eingestellt.

In diese Färbelösung wurde bei 30°C 30 Minuten lang eine Strähne zu 90% ergrautes, nicht vorbehandeltes Menschenhaar eingebracht. Die Strähne wurde mit 30 Sek. mit lauwarmen Wasser gespült, mit warmer Luft getrocknet und anschließend ausgekämmt.

Die jeweiligen Farbnuancen und Farbtiefen sind in der nachfolgenden Tabelle 1 wiedergegeben.

Die Farbtiefe der erhaltenen Färbungen wurde dabei nach folgender Skala bewertet:
- -: : keine oder eine sehr blasse Ausfärbung
- (+): : schwache Intensität
- +: : mittlere Intensität
- +(+): : mittlere bis starke Intensität
- ++: : starke Intensität
- ++(+): : starke bis sehr starke Intensität
- +++: : sehr starke Intensität

**Tabelle 1**

| **Ausfärbungen mit Salicylaldehyd** | | |
|---|---|---|
| Oxidationsfarbstoffvorprodukt | Färbenuance | Farbtiefe |
| 2,4,5,6-Tetraaminopyrimidin x H₂S0₄ | gelborange | ++(+) |
| 1,8-Bis-(2,5-diaminophenoxy)-3,6-dioxaoctan x 4HCl | blauschwarz | +++ |
| 2-Methylamino-3-amino-6-methoxypyridin x 2HCl | gelbbraun | ++(+) |
| 2-(2,5-Diaminophenyl)-ethanol x H₂S0₄ | graubraun | ++ |
| 2-Aminomethyl-4-aminophenol x 2HCl | gelbbraun | +(+) |
| 2,6-Dimethoxy-3,5-diaminopyridin x 2HCl | braunschwarz | +++ |

**Tabelle 2**

| **Ausfärbungen mit 2,4-Dihydroxybenzaldehyd** | | |
|---|---|---|
| Oxidationsfarbstoffvorprodukt | Färbenuance | Farbtiefe |
| 2,4,5,6-Tetraaminopyrimidin x H₂S0₄ | dunkelorange | ++(+) |
| 1,8-Bis-(2,5-diaminophenoxy)-3,6-dioxaoctan x 4HCl | dunkelgrau | ++(+) |
| 2-Methylamino-3-amino-6-methoxypyridin x 2HCl | dunkelgelb | ++(+) |
| 2-(2,5-Diaminophenyl)-ethanol x H₂S0₄ | mittelbraun | ++ |
| 2-Aminomethyl-4-aminophenol x 2HCl | gelb | ++ |
| 2,6-Dimethoxy-3,5-diaminopyridin x 2HCl | braunschwarz | +++ |

**Tabelle 3**

| **Ausfärbungen mit Vanillin** | | |
|---|---|---|
| Oxidationsfarbstoffvorprodukt | Färbenuance | Farbtiefe |
| 2,4,5,6-Tetraaminopyrimidin x H₂S0₄ | orange | ++(+) |
| 1,8-Bis-(2,5-diaminophenoxy)-3,6-dioxaoctan x 4HCl | dunkelgrau | ++(+) |
| 2-Methylamino-3-amino-6-methoxypyridin x 2HCl | gelbbraun | ++ |
| 2-(2,5-Diaminophenyl)-ethanol x H₂S0₄ | orangebraun | ++ |
| 2-Aminomethyl-4-aminophenol x 2HCl | dunkelgelb | ++(+) |
| 2,6-Dimethoxy-3,5-diaminopyridin x 2HCl | olivbraun | ++(+) |

**Tabelle 4**

| **Ausfärbungen mit 2,3,4-Trihydroxybenzaldehyd** | | |
|---|---|---|
| Oxidationsfarbstoffvorprodukt | Färbenuance | Farbtiefe |
| 2,4,5,6-Tetraaminopyrimidin x H₂S0₄ | mittelbraun | +(+) |
| 1,8-Bis-(2,5-diaminophenoxy)-3,6-dioxaoctan x 4HCl | grüngrau | ++(+) |
| 2-Methylamino-3-amino-6-methoxypyridin x 2HCl | braungelb | ++ |
| 2-(2,5-Diaminophenyl)-ethanol x H₂S0₄ | mittelbraun | ++ |
| 2-Aminomethyl-4-aminophenol x 2HCl | braunorange | ++ |
| 2,6-Dimethoxy-3,5-diaminopyridin x 2HCl | schwarz | +++ |

**Tabelle 5**

| **Ausfärbungen mit 2,5-Dihydroxybenzaldehyd** | | |
|---|---|---|
| Oxidationsfarbstoffvorprodukt | Färbenuance | Farbtiefe |
| 2,4,5,6-Tetraaminopyrimidin x H₂S0₄ | hellgelbbraun | + |
| 1,8-Bis-(2,5-diaminophenoxy)-3,6-dioxaoctan x 4HCl | grau | +(+) |
| 2-Methylamino-3-amino-6-methoxypyridin x 2HCl | mittelbraun | + |
| 2-(2,5-Diaminophenyl)-ethanol x H₂S0₄ | mittelbraun | +(+) |
| 2-Aminomethyl-4-aminophenol x 2HCl | hellorange | ++ |
| 2,6-Dimethoxy-3,5-diaminopyridin x 2HCl | dunkelgrau | ++(+) |

**Tabelle 6**

| **Ausfärbungen mit 4-(Dimethylamino)-benzaldehyd** | | |
|---|---|---|
| Oxidationsfarbstoffvorprodukt | Färbenuance | Farbtiefe |
| 2,4,5,6-Tetraaminopyrimidin x H₂S0₄ | tiefgelb | ++ |
| 1,8-Bis-(2,5-diaminophenoxy)-3,6-dioxaoctan x 4HCl | rotbraun | ++(+) |
| 2-Methylamino-3-amino-6-methoxypyridin x 2HCl | gelbbraun | ++(+) |
| 2-(2,5-Diaminophenyl)-ethanol x H₂S0₄ | kupfer | ++ |
| 2-Aminomethyl-4-aminophenol x 2HCl | gelborange | ++ |
| 2,6-Dimethoxy-3,5-diaminopyridin x 2hcl | schwarz | +++ |

**Tabelle 7**

| **Ausfärbungen mit 4-Dimethylaminozimtaldehyd** | | |
|---|---|---|
| Oxidationsfarbstoffvorprodukt | Färbenuance | Farbtiefe |
| 2,5-Diaminotoluol x H₂SO₄ | rotviolett | ++(+) |
| 2,4,5,6-Tetraaminopyrimidin x H₂S0₄ | orange | +++ |
| 1,8-Bis-(2,5-diaminophenoxy)-3,6-dioxaoctan x 4HCl | grünviolett | ++(+) |
| 2-Methylamino-3-amino-6-methoxypyridin x 2HCl | braungelb | ++(+) |
| 2-(2,5-Diaminophenyl)-ethanol x H₂S0₄ | violettrot | +++ |
| 2-Aminomethyl-4-aminophenol x 2HCl | dunkelviolett | +++ |
| N,N-Bis-(2-hydroxyethyl)-p-phenylendiamin x H₂S0₄ | grauviolett | ++(+) |
| 4,4'-diaminodiphenylamin x H₂S0₄ | braunschwarz | +++ |
| 2,6-Dimethoxy-3,5-diaminopyridin x 2HCl | braunschwarz | +++ |
| L-Histidin | goldgelb | ++ |
| L-Tryptophan | goldgelb | ++ |
| L-Tyrosin | goldgelb | ++(+) |
| L-Prolin | goldgelb | ++(+) |
| Pyrrol | goldgelb | ++(+) |

**Tabelle 8**

| **Ausfärbungen mit 4-Hydroxy-3-methoxyzimtaldehyd (Coniferylaldehyd)** | | |
|---|---|---|
| Oxidationsfarbstoffvorprodukt | Färbenuance | Farbtiefe |
| 2,4,5,6-Tetraaminopyrimidin x H₂S0₄ | gelborange | +(+) |
| 2-Methylamino-3-amino-6-methoxypyridin x 2HCl | olivgrün | ++ |
| 2-(2,5-Diaminophenyl)-ethanol x H₂S0₄ | rot | +++ |
| 2-Aminomethyl-4-aminophenol x 2HCl | rot | ++(+) |
| 4,4'-Diaminophenylamin x H₂S0₄ | blaugrün | ++(+) |
| 2,6-Dimethoxy-3,5-diaminopyridin x 2HCl | grünschwarz | +++ |
| N,N-Bis-(2-hydroxyethyl)-p-phenylendiamin. H₂SO₄ | dunkelviolett | ++(+) |
| 2,5-Diaminotoluol x H₂S0₄ | braunrot | ++(+) |

## Patentansprüche

1. Mittel zum Färben von keratinhaltigen Fasern, insbesondere menschlichen Haaren, enthaltend
a) einen Aldehyd der Formel I 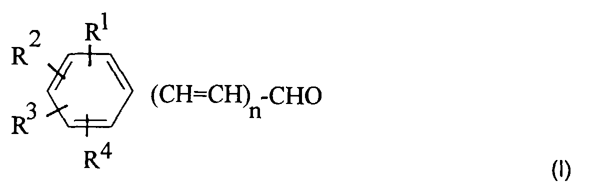 in der R¹, R², R³ und R⁴ unabhängig voneinander für Wasserstoffatome, Halogenatome, C₁-C₄-Alkyl-, C₂-C₄-Hydroxyalkyl-, Amino-C₁-C₄-alkyl-, C₁-C₄-Alk-oxy-, Nitro-, Carboxy-, C₁-C₄-Acylamino-, Sulfo-, Hydroxy- oder Aminogruppen, die durch C₁-C₄-Alkyl- oder C₂-C₄-Hydroxyalkylgruppen substituiert sein können, stehen, und n für 0 oder 1 steht, und
b) ein Oxidationsfarbstoffvorprodukt, das ein primäres oder sekundäres aromatisches Amin; eine stickstoffhaltige heterocyclische Verbindung; eine aromatische Hydroxyverbindung; eine Aminosäure; ein aus 2 bis 9 Aminosäuren aufgebautes Oligopeptid; oder ein Gemisch der voranstehenden ist,
mit der Maßgabe, daß, wenn n gleich 0 ist, das Oxidationsfarbstoffvorprodukt ausgewählt ist aus der Gruppe 2-Aminomethyl-4-aminophenol, 1,8-Bis-(2,5-diaminophenoxy)-3,6-dioxaoctan, 2,4,5,6-Tetraaminopyrimidin, 2-Methylamino-3-amino-6-methoxypyridin, 2,6-Dimethoxy-3,5-diaminopyridin, 2-(2,5-Diaminophenyl)-ethanol und 1,3-Bis-(2,4-diaminophenoxy)-propan, Aminosäuren, aus 2 bis 9 Aminosäuren aufgebauten Oligopeptide, oder Gemischen der voranstehenden.

2. Mittel nach Anspruch 1, dadurch gekennzeichnet, daß die Aldehyde der Formel I und die Oxidationsfarbstoffvorprodukte in einer Menge von jeweils 0,03 bis 65, insbesondere 1 bis 40 mmol, jeweils bezogen auf 100 g des gesamten Färbemittels, enthalten sind.

3. Mittel nach Anspruch 1 oder 2, dadurch gekennzeichnet, daß primäre oder sekundäre aromatische Amin ausgewählt ist aus der Gruppe bestehend aus N-(2-Hydroxyethyl)-N-ethyl-, N-(2-Methoxyethyl-), 2,3-, 2,4-, 2,5-Dichlor-p-phenylendiamin, 2-Chlor-p-phenylendiamin, 2,5-Dihydroxy-4-morpholinoanilin-dihydrobromid, 2-, 3-Aminophenol, o-, m-Phenylendiamin, 2,5-Diaminotoluol, - phenol, -anisol, -phenethol, 2-Chlor-p-phenylendiamin, 4-Methylamino-, 3-, 4-Dimethylamino-, 3,4-Methylendioxyanilin, 3-Amino-2,4-dichlor-, 4-Methylamino-, 2-Methyl-5-amino-, 3-Methyl-4-amino-, 2-Methyl-5-(2-hydroxyethylamino)-, 2-Methyl-5-amino-6-chlor-, 2-Methyl-5-amino-4-chlor-, 2-Methyl-5-amino-6-chlor-, 5-(2-Hydroxyethylamino)-4-methoxy-2-methyl-, 4-Amino-2-aminomethyl-phenol, 1,3-Diamino-2,4-dimethoxybenzol, 2-, 3-, 4-Aminobenzoesäure, -phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxy-benzoesäure, 2-, 3-, 4-Aminobenzolsulfonsäure, 3-Amino-4-hydroxybenzolsulfonsäure, 4-Amino-3-hydroxynaphthalin-Isulfonsäure, 6-Amino-7-hydroxynaphthalin-2-sulfonsäure, 7-Amino-4hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol-tetrahydrochlorid, 2,4,5-Triaminophenol-trihydrochlorid, Pentaaminobenzol-pentahydrochlorid, Hexamaminobenzol-hexahydrochlorid, 2,4,6-Triaminoresorcin-trihydrochlorid, 4,5-Diaminobrenzcatechinsulfat, 4,6-Diaminopyrogallol-dihydrochlorid, 3,5-Diamino-4-hydroxybrenzcatechin-sulfat, aromatische Aniline bzw. Phenole mit einem weiteren aromatischen Rest wie 4,4'-Diaminostilben-dihydrochlorid, 4,4'-Diaminostilben-2,2'-disulfonsäure, Na-Salz, 4,41-Diaminodiphenylmethan, -sulfid, -sulfoxid, -amin, 4,4'-Diaminodiphenylamin-2-sulfonsäure, 4,4'-Diaminobenzophenon, -diphenylether, 3,3',4,4'-Tetraaminodiphenyl-tetrahydrochlorid, 3,3',4,4'-Tetraamino-benzophenon, 1,3-Bis-(2,4-diaminophenoxy)-propan-tetrahydrochlorid, 1,8-Bis-(2,5-diaminophenoxy)-3,6-dioxaoctan-tetrahydrochlorid, 1,3-Bis-(4-aminophenylamino)-propan, -2-propanol, 1,3-Bis-[N-(4-aminophenyl)-2hydroxyethylamino]-2-propanol, N,N-Bis-[2-(4-aminophenoxy)-ethyl]methylamin-trihydrochlorid;
einer stickstoffhaltigen heterocyclischen Verbindung ausgewählt aus der Gruppe aus 2-, 3-, 4-Amino-, 2-Amino-3-hydroxy-, 2,6-Diamino-, 2,5-Diamino-, 2,3-Diamino-, 2-Dimethylamino-5-amino-, 3-Amino-2-methylamino-6-methoxy-, 2,3-Diamino-6-methoxy-, 3,5-Diamino-2,6-dimethoxy-, 2,4,5-Triamino-, 2,6-Dihydroxy-3,4-dimethylpyridin, 4,5,6-Triamino-, 4-Hydroxy-2,5,6-triamino-, 2,4,5,6-Tetraamino-, 2-Methylamino-4,5,6-triamino-, 2,4-, 4,5-Diamino-, 2-Amino-4-methoxy-6-methyl-pyrimidin, 3,5-Diaminopyrazol, -1,2,4-triazol, 3-Amino-, 3-Amino-5-hydroxypyrazol, 2-,3-, 8-Aminochinolin, 4-Amino-chinaldin, 2-, 6-Aminonicotinsäure, 5-Aminoisochinolin, 4-, 5-, 6-, 7-Aminoindol, 5-, 6-Aminoindazol, 5-, 7-Amino-benzimidazol, -benzothiazol, 2,5-Dihydroxy-4-morpholinoanilin sowie Indol- und Indolinderivaten, wie 5,6-Dihydroxyindol, 5,6-Dihydroxyindolin und 4-Hydroxyindolin, deren mit vorzugsweise anorganischen Säuren gebildeten physiologisch verträglichen Salze;

4. Mittel nach einem der Ansprüche 1 bis 3, dadurch gekennzeichnet, daß der Aldehyd der Formel I ausgewählt ist aus der Gruppe 4-Dimethylaminozimtaldehyd, 4-Dimethylaminobenzaldehyd, 2-Hydroxybenzaldehyd (Salicylaldehyd), 4-Hydroxy-3-methoxybenzaldehyd (Vanillin), 2,3,4Trihydroxybenzaldehyd, 4-Diethylamino-2-hydroxybenzaldehyd und 3-Methoxy-4-hydroxyzimtaldehyd (Coniferylaldehyd).

5. Mittel nach einem der Ansprüche 1 bis 4, dadurch gekennzeichnet, daß es direktziehende Farbstoffe in einer Menge von 0,01 bis 20 Gew.-%, bezogen auf das gesamte Oxidationshaarfärbemittel, enthält.

6. Mittel nach einem der Ansprüche 1 bis 5, dadurch gekennzeichnet, daß es Oxidationsmittel in einer Menge von 0,01 bis 6 Gew.-%, bezogen auf die Anwendungslösung, enthält.

7. Mittel nach Anspruch 6, dadurch gekennzeichnet, daß als Oxidationsmittel H₂O₂ enthalten ist.

8. Mittel nach einem der Ansprüche 1 bis 7, dadurch gekennzeichnet, daß es anionische, zwitterionische oder nichtionische Tenside enthält.

9. Verwendung von Aldehyden der Formel I 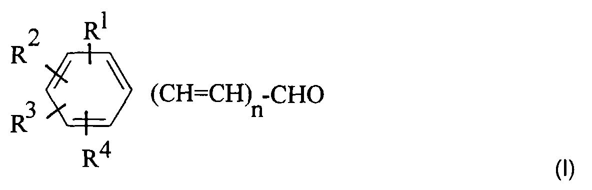 worin R¹, R², R³, R⁴ und n wie in Anpruch 1 definiert sind, als eine färbende Komponente in Oxidationshaarfärbemitteln.

10. Verfahren zum Färben von keratinhaltigen Fasern, worin ein Oxidationsfärbemittel, enthaltend
a) mindestens einen Aldehyd der Formel I 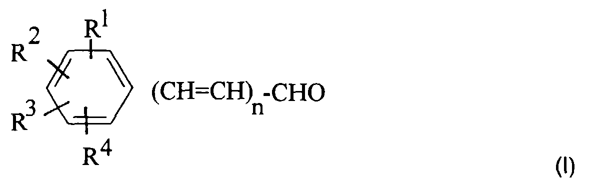 worin R¹, R², R³, R⁴ und n wie in Anspruch 1 definiert sind, und
b) mindestens ein Oxidationsfarbstoffvorprodukt, das ein primäres oder sekundäres aromatisches Amin; eine stickstoffhaltige heterocyclische Verbindung; eine aromatische Hydroxyverbindung; eine Aminosäure; ein aus 2 bis 9 Aminosäuren aufgebautes Oligopeptid; oder ein Gemisch der voranstehenden ist,
mit der Maßgabe, daß, wenn n gleich 0 ist, das Oxidationsfarbstoffvorprodukt ausgewählt ist aus der Gruppe 2-Aminomethyl-4-aminophenol, 1,8-Bis-(2,5-diaminophenoxy)-3,6-dioxaoctan, 2,4,5,6-Tetraaminopyrimidin, 2-Methylamino-3-amino-6-methoxypyridin, 2,6-Dimethoxy-3,5-diaminopyridin, 2-(2,5-Diaminophenyl)-ethanol und 1,3-Bis-(2,4-diaminophenoxy)-propan, Aminosäuren, aus 2 bis 9 Aminosäuren aufgebauten Oligopeptide,
oder Gemischen der voranstehenden, ggf. im Gemisch mit einem wasserlöslichen Träger und weiteren Wirk-, Zusatz- und Hilfsstoffen auf die keratinhaltigen Fasern aufgebracht, einige Zeit, üblichenweise ca. 30 Minuten, auf der Faser belassen und anschließend wieder ausgespült oder mit einem handelsüblichen Haarshampoo ausgewaschen wird.
